# EUROPEAN PATENT APPLICATION

(11) **EP 4 512 885 A1**
(43) Date of publication of application: **26.02.2025**
(21) Application number: 23864345.6
(22) Date of filing: 06.05.2023
(51) Int. Cl.: C12M 1/34, C12M 1/36, C12M 1/00, C12Q 1/6844

(54) **MOLECULAR DETECTION SYSTEM AND DETECTION METHOD THEREFOR**

(30) Priority: 16.09.2022 CN 202211132154
(71) Applicant: Hunan Biometa Intelligent Manufacturing Technology Co., Ltd, Changsha, Hunan 410205 (CN)
(72) Inventor: XIE, Yaping, Changsha, Hunan 410205 (CN); LONG, Zeyu, Changsha, Hunan 410205 (CN); WU, Chang, Changsha, Hunan 410205 (CN); ZENG, Bo, Changsha, Hunan 410205 (CN); DAI, Lizhong, Changsha, Hunan 410205 (CN)
(74) Representative: RGTH
(86) International application number: PCT/CN2023/092565
(87) International publication number: WO 2024/055605

(57) **Abstract**

Disclosed are a molecular detection system and a detection method thereof. During an actual detection operation, firstly, a transport module is controlled to move to a loading station along a first direction, and a kit is loaded on the transport module. Then, the transport module is controlled to move to be between the loading station and an amplification detection station along the first direction. A bin cover is transferred between reagent bins and temporary holding bins, and a sample-containing reagent is transferred between the reagent bins by using a pipetting module, to make a sample be mixed uniformly and reacted with each reagent sequentially to achieve nucleic acid extraction and/or pre-detection processing. Then, the transport module is controlled to move to the amplification detection station along the first direction, and the amplification and detection is performed on the sample in the kit by using an amplification detection module. After the amplification and detection is completed, the transport module is controlled to return to the loading station along the first direction, and the kit is unloaded after detection, so that one detection is completed. This detection process may simplify an operation and improve a detection efficiency.

## Description

### CROSS-REFERENCE TO RELATED APPLICATIONS

The present application claims priority to the Chinese Patent Application No. 202211132154.7, filed on September 16, 2022, and the entire contents of which are hereby incorporated by reference in their entireties.

### TECHNICAL FIELD

The present invention relates to the field of biological detection devices technologies, and in particular, to a molecular detection system and a detection method thereof.

### BACKGROUND

A molecular detection device is used for detecting biological samples, which mainly includes steps of nucleic acid extraction, amplification and detection and the like, and it is necessary to add a sample, and transfer and mix the sample for many times to complete the above-mentioned steps of the nucleic acid extraction, the amplification and the detection and the like.

However, a conventional molecular detection device needs to be separately equipped with an external centrifuge. After a nucleic acid sample is added to a reaction tube, the reaction tube needs to be manually taken out of the molecular detection device and transferred to the external centrifuge for centrifugation, and then the reaction tube is manually transferred to a polymerase chain reaction (Polymerase Chain Reaction, PCR) module for optical detection, so that operations are cumbersome and labor-consuming. In addition, the reaction tube is generally not closed during use, so that there is a possibility of sample pollution.

### SUMMARY

Based on this, it is necessary to provide a molecular detection system and a detection method thereof for overcoming defects above-mentioned to solve problems of cumbersome in operation and a risk of sample pollution in a conventional molecular detection technology.

According to a first aspect of the present invention, a molecular detection system is provided, which includes a molecular detection device and a kit. The kit includes a plurality of reagent bins, a plurality of bin covers configured to cover the plurality of reagent bins in a one-to-one correspondence manner, a plurality of temporary holding bins configured to temporarily hold the plurality of bin covers, and the molecular detection device has a loading station and an amplification detection station arranged at intervals along a first direction and includes: a transport module is controlled to move between the loading station and the amplification detection station along the first direction; and the transport module being configured to take or unload the kit when moving to the loading station; a pipetting module, arranged corresponding to a position between the loading station and the amplification detection station, and configured to transfer the bin covers between the reagent bins and the temporary holding bins and transfer a sample-containing reagent between the reagent bins when the transport module moves between the loading station and the amplification detection station; and an amplification detection module, arranged corresponding to the amplification detection station, and configured to perform amplification and detection on a sample solution in the kit when the transport module moves to the amplification detection station.

Optionally, the pipetting module is provided with a connecting part configured to connect to or disconnect from a tip, during a process that the transport module moves between the loading station and the amplification detection station, the connecting part is capable of alternatively aligning with the plurality of reagent bins in a second direction, and the second direction intersects with the first direction; when the transport module moves until the connecting part aligns with any one of the reagent bins in the second direction, the pipetting module is controlled to move in the second direction to drive the connecting part to pick up the bin cover to open the reagent bin or release the bin cover to cover the reagent bin; and when the transport module moves until the connecting part aligns with any one of the temporary holding bins in the second direction, the pipetting module is controlled to move in the second direction to drive the connecting part to release the bin cover to temporarily hold the bin cover in the temporary holding bin or pick up the bin cover from the temporary holding bin.

Optionally, when the transport module moves until the connecting part aligns with any one of the reagent bins opened in the second direction, and the pipetting module is controlled to move in the second direction to drive the tip on the connecting part to be inserted into or withdrawn from a current reagent bin.

Optionally, during the process that the transport module moves between the loading station and the amplification detection station, the connecting part is capable of aligning with a tip bin of the kit for pre-loading the tip in the second direction; and when the transport module moves until the connecting part aligns with the tip bin in the second direction, the pipetting module is controlled to move in the second direction to drive the connecting part to be inserted into or withdrawn from the tip bin, to make the connecting part pick up the tip in the tip bin or releases the tip on the connecting part to the tip bin.

Optionally, during the process that the transport module moves between the loading station and the amplification detection station, the connecting part is capable of alternatively aligning with an injection bin of the kit and a plunger bin configured to load a plunger in the second direction; when the transport module moves until the connecting part aligns with the plunger bin in the second direction, the pipetting module is controlled to move in the second direction to drive the connecting part to be inserted into or withdrawn from the plunger bin to pick up the plunger; and when the transport module moves until the connecting part aligns with the injection bin in the second direction, the pipetting module is controlled to move in the second direction to drive the plunger on the connecting part to be inserted into the injection bin to inject a sample-containing reagent in the injection bin into a reaction tube of the kit.

Optionally, when the transport module moves to the amplification detection station, the reaction tube of the kit is fitted with and connected to the amplification detection module, and the amplification detection module is configured to perform amplification and detection on the sample-containing reagent in the reaction tube of the kit.

Optionally, the molecular detection device further includes a first driving module, and the first driving module is in driving connection with the pipetting module to drive the pipetting module to move in the second direction.

Optionally, the first driving module includes a first mounting base, a first driving member, a driving wheel, a driven wheel and a transmission belt; the pipetting module is movably connected to the first mounting base in the second direction, the first driving member is mounted on the first mounting base and is configured to be electrically connected to a communication module, the driving wheel is in transmission connection with an output shaft of the first driving member, the driven wheel is rotatably connected to the first mounting base and is arranged at intervals with the driving wheel in the second direction, and the transmission belt is sleeved between the driving wheel and the driven wheel and is fixedly connected to the pipetting module.

Optionally, the molecular detection device further includes a second driving module, and the second driving module is in driving connection with the transport module to drive the transport module to move along the first direction.

Optionally, the second driving module includes a second mounting base, a second driving member, a lead screw and a lead screw nut; the transport module is movably connected to the second mounting base in the first direction, the lead screw is rotatably connected to the second mounting base around an axis of the lead screw, the axis of the lead screw is parallel to the first direction, the second driving member is in transmission connection with the lead screw and is configured to be electrically connected to the communication module, and the lead screw nut is threadedly connected to the lead screw and is fixedly connected to the transport module.

According to a second aspect of the present invention, a detection method applied to the above-mentioned molecular detection system is provided, which includes following steps: a, moving the transport module to the loading station along a first direction, and loading the kit on the transport module, where the kit is pre-loaded with a sample, various reagents for nucleic acid extraction and pretreatment before detection; b, moving the transport module to be between a loading station and an amplification detection station along the first direction, and transferring, by the pipetting module, the bin cover between the reagent bin and the temporary holding bin and the sample between each of the reagent bins, to make the sample be mixed uniformly and reacted with each of the reagents sequentially; and c, moving the transport module to the amplification detection station along the first direction, and performing, by the amplification detection station, amplification and detection on a sample-containing reagent in the kit.

Through the above-mentioned technical solutions, during an actual detection operation, firstly, the transport module is controlled to move to the loading station along the first direction, the kit is loaded on the transport module, and the sample to be detected and various reagents are pre-loaded in the kit. Then, the transport module is controlled to move to be between the loading station and the amplification detection station along the first direction, and the bin cover is transferred between the reagent bin and the temporary holding bin and a sample-containing reagent is transferred between the reagent bins by using the pipetting module, to make the sample be mixed uniformly and reacted with each of the reagents sequentially to achieve nucleic acid extraction and/or pre-detection processing. Then, the transport module is controlled to move to the amplification detection station along the first direction, and the amplification and detection is performed on the sample in the kit by using the amplification detection module. After the amplification and detection is completed, the transport module is controlled to return to the loading station along the first direction, and the kit is unloaded after detection, so that one detection is completed.

It may be seen that, a centrifugal processing is not required in a whole process of the detection above-mentioned, so that manual operation is saved. The molecular detection device may realize sample-in and result-out, so that an operation is simplified and a detection efficiency is improved. In addition, since the reagent bin may be covered by a corresponding bin cover, so that a risk of sample pollution may be greatly reduced, and thus an accuracy of the detection result is improved.

### BRIEF DESCRIPTION OF THE DRAWINGS

The drawings are used to provide a further understanding of the present invention and constitute a portion of the specification, and together with the following detailed description, serve to explain the present invention, but do not constitute a limitation to the present invention. In the drawings:
FIG. 1 is a schematic structural diagram (omitting a box body) of a molecular detection device according to a specific embodiment of the present invention.
FIG. 2 is a front view of the molecular detection device shown in FIG. 1.
FIG. 3 is a schematic structural diagram of a kit according to an embodiment of the present invention.
FIG. 4 is a front view of a first driving module of the molecular detection device shown in FIG. 1.
FIG. 5 is a schematic structural diagram of a second driving module and a transport module of the molecular detection device shown in FIG. 1.
FIG. 6 is a schematic structural diagram of a kit provided with a bin cover according to an embodiment of the present invention.
FIG. 7 is a schematic assembly diagram of a reagent bin and the bin cover shown in FIG. 6.
FIG. 8 is a flowchart of specific steps of a molecular detection method according to a specific embodiment of the present invention;
FIG. 9 is a flowchart of specific steps of a step S20 in the molecular detection method shown in FIG. 8.

### DETAILED DESCRIPTION OF THE EMBODIMENTS

In order to make the above-mentioned objectives, features and advantages of the present invention more apparent and easier to be understood, specific embodiments of the present invention are described in detail below with reference to the drawings. In the following description, numerous specific details are set forth for thoroughly understanding the present invention. However, the present invention can be implemented in many other manners different from those described herein, and a person skilled in the art may make similar improvements without departing from the connotation of the present invention, and therefore, the present invention is not limited by the specific embodiments disclosed below.

In the description of the present invention, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", and the like are merely intended to facilitate describing the present invention and simplify the description, rather than indicating or implying that the device or element referred to must have a specific orientation or be constructed and operated in a specific orientation, and thus it cannot be understood as a limitation to the present invention.

In addition, the terms "first" and "second" are merely used for descriptive purposes and cannot be understood as indicating or implying relative importance or implicitly indicating the number of the indicated technical features. Consequently, the features defined with the "first" and "second" may explicitly or implicitly include at least one of the features. In the description of the present invention, "a plurality of" means at least two, for example, two, three, and the like, unless specifically defined otherwise.

In the present invention, unless otherwise specified and limited, terms such as "mount", "communicate", "connect", and "fix" should be understood in a broad sense, for example, it may be a fixed connection, a detachable connection, or an integration; it may be a mechanical connection or an electrical connection; it may be directly connected or indirectly connected by using an intermediate medium; and it may be the internal communication between two elements or an interaction relationship between two elements unless expressly limited otherwise. For a person with ordinary skill in the art, the specific meanings of the above terms in the present invention may be understood according to specific situations.

In the present invention, unless otherwise specified and limited, a first feature being "on" or "under" a second feature may be that the first feature is in direct contact with the second feature, or the first feature is in indirect contact with the second feature through an intermediate medium. Moreover, the first feature being "above", "upper" and "upside" the second feature may mean that the first feature is directly above or obliquely above the second feature, or simply mean that the level of the first feature is higher than that of the second feature. The first feature being "below", "under" and "beneath" the second feature may mean that the first feature is directly below or obliquely below the second feature, or merely mean that a horizontal height of the first feature is less than that of the second feature.

It should be noted that when an element is referred to as being "fixed to" or "disposed on" another element, it may be directly on another element or an intervening element may also exist. When an element is considered to be "connected" to another element, it may be directly connected to another element or an intervening element may exist at the same time. As used herein, the terms "vertical", "horizontal", "upper", "lower", "left", "right", and the like are only for purposes of illustration and are not intended to be the only manner to be implemented.

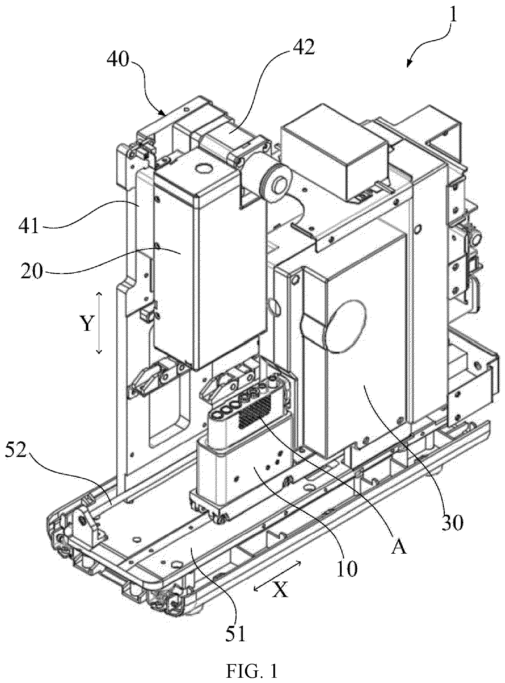

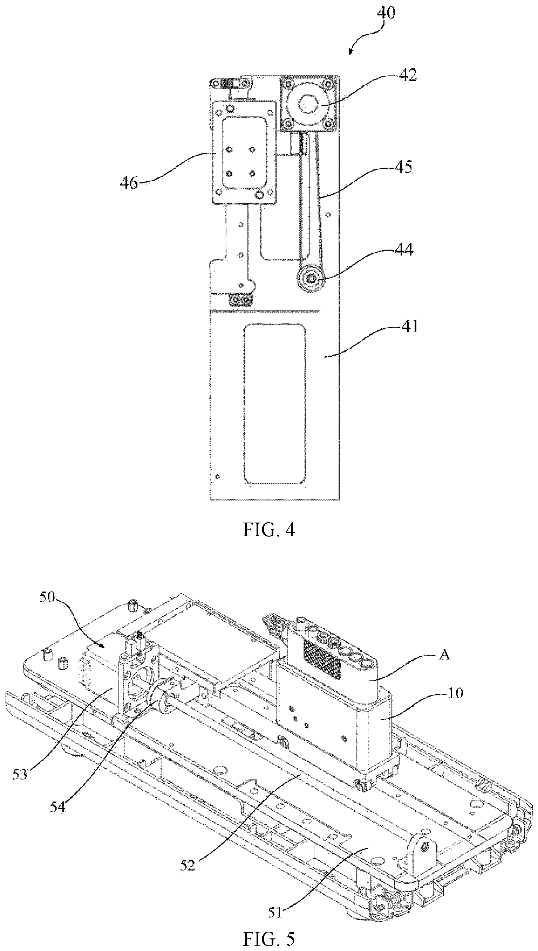

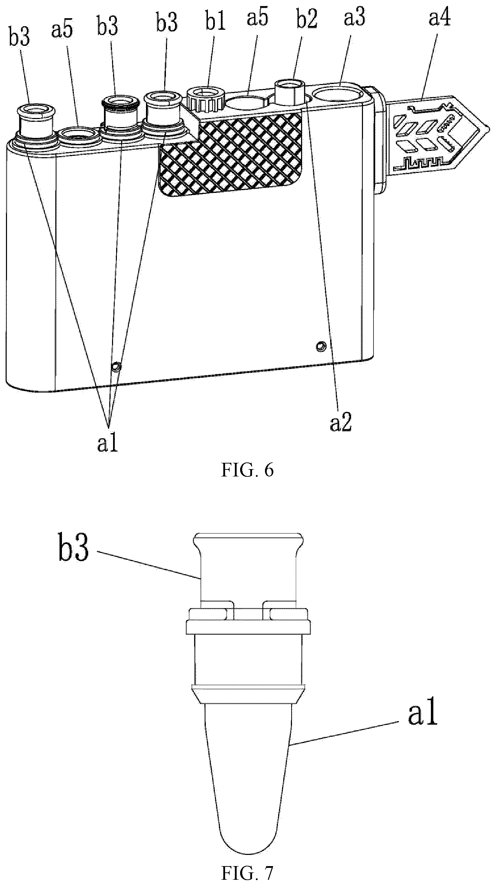

Referring to FIG. 1 to FIG. 7, according to a first embodiment of the present invention, a molecular detection system is provided, which includes a molecular detection device 1 and a kit A.

The kit A includes a plurality of reagent bins a1, a plurality of bin covers b3 configured to cover the plurality of reagent bins a1 in a one-to-one correspondence manner, and a plurality of temporarily-holding bins a5 configured to temporarily hold the plurality of bin covers b3. When the bin cover b3 covers the reagent bin a1, pollution caused by a fact that external pollutants enter the reagent bin a1 may be avoided, and when the bin cover b3 is withdrawn to open the reagent bin a1, the bin cover b3 may be temporarily held on any one of the temporary holding bins a5 for temporarily holding, and when the bin cover b3 needs to be re-covered, the bin cover b3 is taken out from the temporarily-holding bin a5.

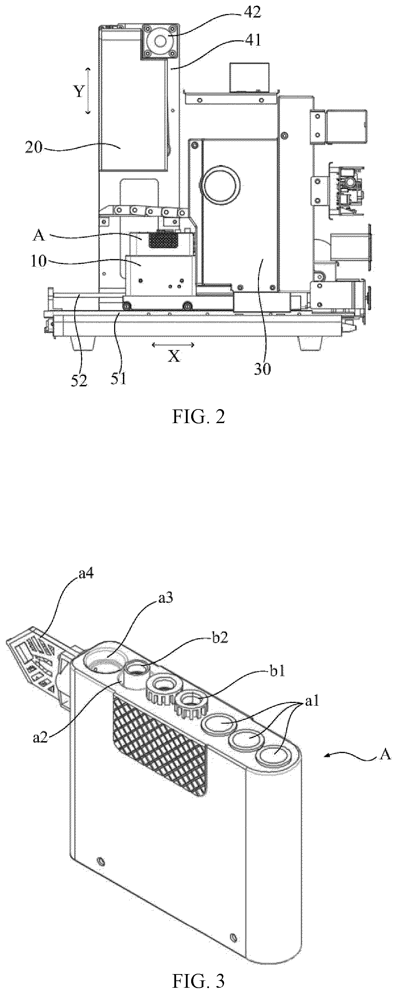

The molecular detection device 1 is provided with a loading station (that is, a left end in FIG. 2) and an amplification detection station (that is, a position shown by a transport module 10 in FIG. 2) arranged at intervals along a first direction X. The molecular detection device 1 includes a transport module 10, a pipetting module 20, and an amplification detection module 30.

The transport module 10 may be controlled to move between the loading station and the amplification detection station along the first direction X. The transport module 10 is configured to take or unload the kit A when moving to the loading station. The kit A is pre-loaded with a sample to be detected and reagents that are used for reacting with the sample. The pipetting module 20 is arranged corresponding to a position between the loading station and the amplification detection station, and is configured to transfer the bin covers b3 between the reagent bins a1 and the temporary holding bins a5 and transfer a sample-containing reagent between the reagent bins a1 when the transport module 10 moves between the loading station and the amplification detection station, so that the sample and each reagent are mixed uniformly and reacted to realize nucleic acid extraction and/or pre-detection processing. The amplification detection module 30 is arranged corresponding to the amplification detection station, and is configured to perform amplification and detection on the sample-containing reagent in the kit A when the transport module 10 moves to the amplification detection station.

According to the molecular detection device 1 mentioned above, during an actual detection operation, firstly, the transport module 10 is controlled to move to the loading station along the first direction X, and the kit A is loaded on the transport module 10, where the sample to be detected and various reagents are pre-loaded in the kit. Then, the transport module 10 is controlled to move to be between the loading station and the amplification detection station along the first direction X, and by using the pipetting module 20, the bin cover b3 is transferred between the reagent bins a1 and the temporary holding bins a5 and the sample-containing reagent is transferred between each of the reagent bins a1, to make the sample is mixed uniformly and reacted with the various reagents sequentially to achieve the nucleic acid extraction and/or the pre-detection processing. Then, the transport module 10 is controlled to move to the amplification detection station along the first direction X, and the amplification and detection is performed on the sample in the kit A by using the amplification detection module 30. After the amplification and detection is completed, the transport module 10 is controlled to return to the loading station along the first direction X, the kit A is unloaded after the detection is completed, and thereby one detection is completed.

In this way, according to the molecular detection device 1 above mentioned, the transport module 10 loading the kit A moves between the loading station and the amplification detection station along the first direction X, so that the nucleic acid extraction, the pre-detection processing and the amplification and detection may be completed. Compared conventional technologies that a card box positioning module, a PCR photoelectric detection and recording module, a magnetic attraction module, a mixing module, a transport module and the like need to move or rotate separately, a movement process is greatly simplified, which is beneficial to simplifying an equipment structure, so that an occupied space is reduced, and thus design requirements of integration and miniaturization are better met.

In addition, a centrifugal processing is not required in a whole process of the detection above-mentioned, so that a manual operation is saved. Sample in and result out may be realized by the molecular detection device 1, so that an operation is simplified, and thus a detection efficiency is improved. In addition, since the reagent bin a1 may be covered by a corresponding bin cover b3, so that a risk of sample pollution may be greatly reduced, and thus an accuracy of a detection result is improved.

In an embodiment of the present invention, the pipetting module 20 is provided with a connecting part (not shown in the figures) configured to connect to or disconnect from a tip b1. During a process that the transport module 10 moves between the loading station and the amplification detection station, the connecting part of the pipetting module 20 may alternately align with the plurality of reagent bins a1 of the kit A for pre-loading a sample or reagents in the second direction Y The second direction Y intersects with the first direction X. Preferably, the second direction Y is perpendicular to the first direction X.

The transfer of the bin cover b3 is realized by cooperation of the transport module 10 and the pipetting module 20.

When the transport module 10 moves until the connecting part aligns with any one of the reagent bins a1 in the second direction Y, the pipetting module 20 is capable of moving along the second direction Y to drive the connecting part to pick up the bin cover b3 to open this reagent bin a1. Then, when the transport module 10 moves until the connecting part picked up the bin cover b3 aligns with any one of the temporary holding bins a5 in the second direction Y, the pipetting module 20 is capable of moving along the second direction Y to drive the connecting part to release the bin cover b3 to temporarily hold the bin cover b3 in the temporary holding bin a5.

When the reagent bin a1 needs to be re-covered, the transport module 10 moves until the connecting part aligns with the temporary holding bin a5 on which the bin cover b3 is held in the second direction Y, and the pipetting module 20 is capable of moving along the second direction Y to pick up the bin cover b3 from the temporary holding bin a5. Then, when the transport module 10 moves until the connecting part picked up the bin cover b3 aligns with the reagent bin a1 required to be covered in the second direction Y, the pipetting module 20 is capable of moving along the second direction Y to release the bin cover b3 to re-cover the reagent bin a1.

It may be seen that, under a condition that the bin cover b3 is provided to prevent the reagent bin a1 from being polluted, the bin cover b3 is transferred between the reagent bin a1 and the temporary holding bin a5 through a cooperative movement of the transport module 10 and the pipetting module 20, so that a normal function of the molecular detection device 1 is not affected may be ensured.

In a specific embodiment, when the transport module 10 moves until the connecting part aligns with any one of the reagent bins a1 in the second direction Y, the pipetting module 20 may be controlled to move along the second direction Yto drive the tip b1 on the connecting part to be inserted into or withdrawn from the current reagent bin a1. In this way, during an actual operation, the transport module 10 is controlled to move along the first direction X until the connecting part aligns with the reagent bin a1 pre-loaded with a sample in the second direction Y, and the pipetting module 20 is controlled to move towards a current reagent bin a1 along the second direction Y, to drive the tip b1 on the connecting part to be inserted into the current reagent bin a1 and suck the sample. After the suction is completed, the pipetting module 20 is controlled to move away from the reagent bin a1 along the second direction Y, to drive the tip b1 on the connecting part to be withdrawn from the current reagent bin a1.Then, the transport module 10 is controlled to move along the first direction X until the connecting part aligns with another reagent bin a1 in the second direction Y, and the pipetting module 20 is controlled to move towards a current reagent bin a1 along the second direction Y, to drive the tip b1 on the connecting part to be inserted into the current reagent bin a1 and inject the sucked sample into the current reagent bin a1, to make the sample be uniformly mixed and reacted with the reagent in the current reagent bin a1. The foregoing processes can be repeated many times in a same manner to make the sample and n be uniformly mixed and reacted with each of the reagent solutions to complete nucleic acid extraction and/or pre-detection processing.

It should be noted that a current reagent bin a1 refers to a reagent bin a1 aligning with the connecting part in the second direction Y In order to make the sample be better mixed uniformly with the reagent in the current reagent bin a1, the pipetting module 20 may be controlled to repeatedly suck and inject for many times to achieve uniform mixing.

In a specific embodiment, during a process that the transport module 10 moves between the loading station and the amplification detection station, the connecting part may align with a tip bin of the kit A configured to pre-load the tip b1 in the second direction Y

When the transport module 10 moves until the connecting part aligns with the tip bin in the second direction Y, the pipetting module 20 may be controlled to move along the second direction Y to drive the connecting part to be inserted into or withdrawn from the tip bin, to make the connecting part pick up the tip b1 in the tip bin or release the tip b1 on the connecting part into the tip bin.

In this way, before a sample transfer is performed, the connecting part needs to pick up the tip b1, and at this time, the transport module 10 is controlled to move along the first direction X until the connecting part aligns with the tip bin in the second direction Y Then, the pipetting module 20 is controlled to move towards the tip bin along the second direction Y until the connecting part is inserted into the tip bin and connected to the tip b1. The pipetting module 20 is controlled to move away from the tip bin along the second direction Y until the connecting part drives the tip b1 to withdraw from the tip bin. Then, the sample-containing reagent is transferred according to the above-mentioned manner.

After the sample-containing reagent is transferred, the used tip b1 needs to be released in the tip bin (avoiding mutual pollution between different reagents), and at this time, the transport module 10 is controlled to move along the first direction X until the connecting part aligns with the tip bin in the second direction Y. Then, the pipetting module 20 is controlled to move towards the tip bin along the second direction Y until the tip b1 is inserted into the tip bin and separated from the connecting part. Then, the pipetting module 20 is controlled to move away from the tip bin along the second direction Y until the connecting part withdraws from the tip bin (the tip b1 is remained in the tip bin). At this point, if the sample-containing reagent needs to be transferred again, an unused tip b1 in another tip bin may be picked up in the same manner, and then the sample-containing reagent is transferred.

In a specific embodiment, during the process that the transport module 10 moves between the loading station and the amplification detection station, the connecting part may alternatively align with an injection bin a3 of the kit A and a plunger bin a2 configured to load a plunger b2 in the second direction Y

When the transport module 10 moves along the first direction X until the connecting part aligns with the plunger bin a2 in the second direction Y, the pipetting module 20 may be controlled to move along the second direction Y to drive the connecting part to be inserted into or withdrawn from the plunger bin a2 to pick up the plunger b2 in the plunger bin a2. When the transport module 10 moves along the first direction X until the connecting part aligns with the injection bin a3 in the second direction Y, the pipetting module 20 may be controlled to move along the second direction Y to drive the plunger b2 on the connecting part to be inserted into the injection bin a3 and move along the injection bin a3, to inject the sample-containing reagent in the injection bin a3 into a reaction tube a4 of the kit A, to facilitate subsequent amplification and detection of the sample-containing reagent in the reaction tube a4.

Further, when the transport module 10 moves to the amplification detection station, the reaction tube a4 of the kit A is fitted with and connected to the amplification detection module 30, and the amplification detection module 30 is configured to perform amplification and detection on the sample-containing reagent in the reaction tube a4.

In this way, after the sample is mixed uniformly and reacted with each reagent and transferred into the injection bin a3 of the kit A, the transport module 10 moves along the first direction X until the connecting part aligns with the plunger bin a2 in the second direction Y. Then, the pipetting module 20 moves towards the plunger bin a2 along the second direction Y until the connecting part is connected with the plunger b2 in the plunger bin a2. The pipetting module 20 moves away from the plunger bin a2 along the second direction Y, to drive the plunger b2 to withdraw from the plunger bin a2 (that is, picking up of the plunger b2 by the connecting part is completed). Then, the transport module 10 moves along the first direction X until the connecting part aligns with the injection bin a3 in the second direction Y The pipetting module 20 moves towards the injection bin a3 along the second direction Y to drive the plunger b2 to be inserted into the injection bin a3 and move along the injection bin a3 until the sample-containing reagent in the injection bin a3 is injected into the reaction tube a4 of the kit A. Then, the pipetting module 20 moves away from the injection bin a3 along the second direction Y, and the connecting part is separated from the plunger b2 and withdrawn from the injection bin a3 (the plunger b2 is remained in the injection bin a3). The transport module 10 moves along the first direction X to the amplification detection station, to make the reaction tube a4 of the kit A is fitted with and connected to the amplification detection module 30. At this time, the amplification detection module 30 performs amplification and detection on the sample-containing reagent in the reaction tube a4 of the kit A.

It may be understood that, the amplification detection module 30 is configured to heat, insulate, and cool the reaction tube a4, to make the reagent-containing reagent in the reaction tube a4 is amplified, and the amplification detection module 30 is further configured to perform fluorescence detection on the sample-containing reagent in the reaction tube a4.

In an embodiment of the present invention, the molecular detection device 1 further includes a first driving module 40. The first driving module 40 is in driving connection with the pipetting module 20 to drive the pipetting module 20 to move along the second direction Y, to facilitate the pipetting module 20 completing actions such as picking up or releasing the tip b 1, sucking or injecting the sample-containing reagent, picking up or releasing the plunger b2, and injecting the sample-containing reagent in the injection bin a3 into the reaction tube a4 by using the plunger b2. The first driving module 40 is electrically connected to a communication module, to make a main control device 2 control the first driving module 40 through the communication module.

In a specific embodiment, the first driving module 40 includes a first mounting base 41, a first driving member 42, a driving wheel (not shown in the figures), a driven wheel 44 and a transmission belt 45. The pipetting module 20 is movably connected to the first mounting base 41 in the second direction Y The first driving member 42 is mounted on the first mounting base 41, and the driving wheel is in transmission connection with an output shaft of the first driving member 42, to make the driving wheel synchronously rotate with the output shaft of the first driving member 42. The driven wheel 44 is rotatably connected to the first mounting base 41 and is arranged at intervals with the driving wheel along the second direction Y The transmission belt 45 is sleeved between the driving wheel and the driven wheel 44, and is fixedly connected to the pipetting module 20, to make the transmission belt 45 be capable of driving the pipetting module 20 to reciprocate along the second direction Y. The first driving member 42 is electrically connected to the communication module, to make the main control device 2 control the first driving member 42 through the communication module.

In this way, when the pipetting module 20 needs to be driven to move along the second direction Y, the first driving member 42 drives the driving wheel to rotate, thereby driving the driving belt 45 to move forward between the driving wheel and the driven wheel 44 in sequence, and thus the driving belt 45 drives the pipetting module 20 to reciprocate along the second direction Y. Optionally, the first driving member 42 may be a motor. It should be noted that, in the present embodiment, movement of the pipetting module 20 along the second direction Y is implemented in a belt transmission manner. Certainly, in other embodiments, other transmission manners may also be adopted, for example, a cylinder, a lead screw, and the like, which is not limited herein.

Further, the pipetting module 20 is provided with a first sliding block 46, the first mounting base 41 is provided with a first sliding rail (not shown in the figures) extending longitudinally along the second direction Y, and this first sliding block 46 is slidably fitted with the first sliding rail. In this way, the movement of the pipetting module 20 relative to the first mounting base 41 along the second direction Y is guided by using the movement of the first sliding block 46 along the first sliding rail.

In an embodiment of the present invention, the molecular detection device 1 further includes a second driving module 50, and the second driving module 50 is in driving connection with the transport module 10 to drive the transport module 10 to move along the first direction X, so that the transport module 10 reciprocates between the loading station and the amplification detection station, and thus cooperating with the pipetting module 20 and the amplification detection module 30 to complete nucleic acid extraction, pre-detection processing, and amplification and detection. The second driving module 50 is electrically connected to the communication module, to make the main control device 2 control the second driving module 50 through the communication module.

In a specific embodiment, the second driving module 50 includes a second mounting base 51, a second driving member 53, a lead screw 52, and a lead screw nut 54.

The transport module 10 is movably connected to the second mounting base 51 in the first direction X. The lead screw 52 is rotatably connected to the second mounting base 51 around an axis of the lead screw 52, and the axis of the lead screw 52 is parallel to the first direction X. The second driving member 53 is in driving connection with the lead screw 52 to drive the lead screw 52 to rotate around the axis of the lead screw 52. The lead screw nut 54 is threadedly connected to the lead screw 52, and is fixedly connected to the transport module 10. The second driving member 53 is electrically connected to the communication module, to make the main control device 2 control the second driving member 53 through the communication module.

In this way, when the transport module 10 needs to be controlled to move along the first direction X, the second driving member 53 drives the lead screw 52 to rotate around the axis of the lead screw 52, so that the lead screw nut 54 is driven to move in an axial direction (that is, the first direction X) of the lead screw 52, and thus the lead screw nut 54 drives the transport module 10 to move along the first direction X. Optionally, the second driving member 53 may be a motor.

In an embodiment of the present invention, the molecular detection device 1 further includes a case 60, and the transport module 10, the pipetting module 20 and the amplification detection module 30 are all accommodated in the case 60. In this way, the transport module 10, the pipetting module 20 and the amplification detection module 30 are integrally disposed in the case 60, so that a function of sample-in and result-out is realized, which is beneficial to meet a design requirement of integration.

In a specific embodiment, the case 60 has an opening (not shown in the figures), and when the transport module 10 moves to the loading station, a carrying position for carrying the kit A extends out of the case 60 from the opening (that is, out of the case), to unload the kit A on the carrying position or load the kit A on the carrying position. Further, a bin door 61 is mounted at the opening of the case 60. During the transport module 10 moving to the loading station along the first direction X, the bin door 61 is opened, to facilitate the carrying position of the transport module 10 extending out of the case 60 through the opening. During the transport module 10 moving from the loading station to the amplification detection station along the first direction X, the bin door 61 is closed, that is, the opening of the case 60 is closed. It should be noted that the bin door 61 may be controlled to open and close in a separate control manner, and certainly, the bin door 61 may also be controlled in a linkage manner with the transport module 10, which is not limited herein.

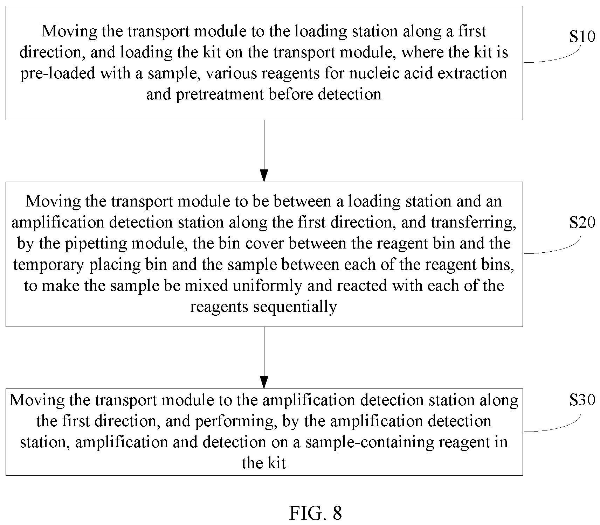

Please refer to FIG. 8, a second embodiment of the present invention provides a detection method using the molecular detection system above-mentioned, which includes following steps:

S10, moving a transport module 10 to a loading station along a first direction X, and loading a kit A on the transport module 10, where the kit A is pre-loaded with a sample and various reagents configured for nucleic acid extraction and/or pretreatment before detection.

S20, moving the transport module 10 to be located between the loading station and an amplification detection station along the first direction X, and transferring, a bin cover b3 between a reagent bin a1 and a temporary holding bin a5 and the sample between each of the reagent bins a1 through the pipetting module 20, to make the samples be mixed uniformly and reacted with each of the reagents sequentially;

S30: moving the transport module 10 to the amplification detection station along the first direction X, and performing amplification and detection on a sample-containing reagent in the kit A through the amplification detection module 30.

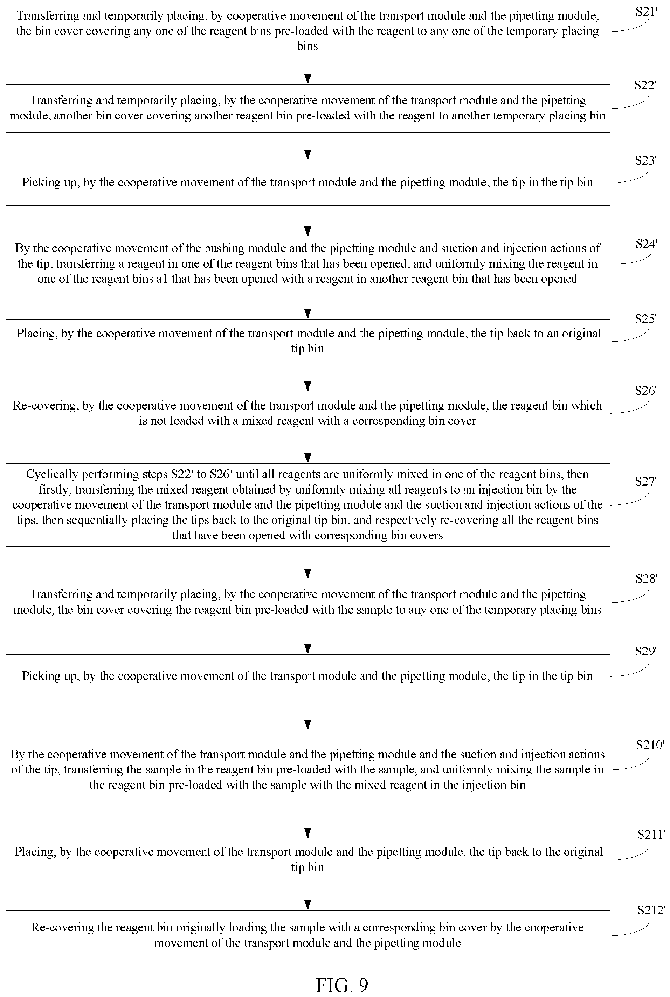

Please refer to FIG. 9, in a specific embodiment, step S20 may further include following steps:
S21', transferring and temporarily holding, by cooperative movement of the transport module 10 and the pipetting module 20, the bin cover b3 covering any one of the reagent bins a1 pre-loaded with the reagent to any one of the temporary holding bins a5;
S22', transferring and temporarily holding, by the cooperative movement of the transport module 10 and the pipetting module 20, another bin cover b3 covering another reagent bin a1 pre-loaded with the reagent to another temporary holding bin a5;
S23', picking up, by the cooperative movement of the transport module 10 and the pipetting module 20, the tip b1 in the tip bin;
S24', by the cooperative movement of the pushing module 10 and the pipetting module 20 and suction and injection actions of the tip b1, transferring a reagent in one of the reagent bins a1 that has been opened, and uniformly mixing the reagent in one of the reagent bins a1 that has been opened with a reagent in another reagent bin a1 that has been opened;
S25', holding, by the cooperative movement of the transport module 10 and the pipetting module 20, the tip b1 back to an original tip bin;
S26', re-covering, by the cooperative movement of the transport module 10 and the pipetting module 20, the reagent bin a1 which is not loaded with a mixed reagent with a corresponding bin cover b3;
S27', cyclically performing steps S22' to S26' until all reagents are uniformly mixed in one of the reagent bins a1, then firstly, transferring the mixed reagent obtained by uniformly mixing all reagents to an injection bin a3 by the cooperative movement of the transport module 10 and the pipetting module 20 and the suction and injection actions of the tips b1, then sequentially holding the tips b1 back to the original tip bin, and respectively re-covering all the reagent bins a1 that have been opened with corresponding bin covers b3;
S28', transferring and temporarily holding, by the cooperative movement of the transport module 10 and the pipetting module 20, the bin cover b3 covering the reagent bin a1 pre-loaded with the sample to any one of the temporary holding bins a5;
S29', picking up, by the cooperative movement of the transport module 10 and the pipetting module 20, the tip b1 in the tip bin;
S210', by the cooperative movement of the transport module 10 and the pipetting module 20 and the suction and injection actions of the tip b1, transferring the sample in the reagent bin a1 pre-loaded with the sample, and uniformly mixing the sample in the reagent bin a1 pre-loaded with the sample with the mixed reagent in the injection bin a3;
S211', holding, by the cooperative movement of the transport module 10 and the pipetting module 20, the tip b1 back to the original tip bin; and
S212', re-covering the reagent bin a1 originally loading the sample with a corresponding bin cover b3 by the cooperative movement of the transport module 10 and the pipetting module 20.

Further, after step S212', the detection method further includes the following steps:
S213', moving the transport module 10 along the first direction X until the connecting part aligns with the plunger bin a2 of the kit A in the second direction Y;
S214', reciprocating the pipetting module 20 along the second direction Y to drive the connecting part to be inserted firstly and then drawn from a current plunger bin a2, to make the connecting part pick up the plunger b2 in a current plunger bin a2;
S215', moving the transport module 10 along the first direction X until the connecting part aligns with the injection bin a3 of the kit A in the second direction Y;
S216', moving the pipetting module 20 towards the injection bin a3 along the second direction Y to drive the plunger b2 on the connecting part to be inserted into the injection bin a3 and move along the injection bin a3 until a sample-containing reagent in the injection bin a3 is injected into a reaction tube a4 of the kit A;
S217', moving the pipetting module 20 away from the injection bin a3 along the second direction Y to drive the connecting part to be separated from the plunger b2 and withdrawn from the injection bin a3.

The technical features of the above embodiments may be combined arbitrarily. To make the description concise, not all possible combinations of the technical features in the above-mentioned embodiments are described. However, all of the combinations of these technical features should be considered as being fallen within the scope of the present invention, as long as such combinations do not contradict with each other.

The above-mentioned embodiments merely illustrate several embodiments of the present invention, and the description thereof is relatively specific and detailed, but cannot be understood as a limitation to the protection scope of the present invention. It should be noted that, for a person of ordinary skill in the art, several modifications and improvements may be made without departing from the concept of the present invention, which all fall within the protection scope of the present invention. Therefore, the protection scope of the present invention shall be subject to the appended claims.

## Claims

1. A molecular detection system, comprising a molecular detection device (1) and a kit (A), wherein the kit (A) comprises a plurality of reagent bins (a1), a plurality of bin covers (b3) configured to cover the plurality of reagent bins (a1) in a one-to-one correspondence manner and a plurality of temporary holding bins (a5) configured to temporarily hold the plurality of bin covers (b3), and the molecular detection device (1) has a loading station and an amplification detection station arranged at intervals along a first direction (X) and comprises:
a transport module (10) is controlled to move between the loading station and the amplification detection station along the first direction (X), and the transport module (10) being configured to take or unload the kit (A) when moving to the loading station;
a pipetting module (20), arranged corresponding to a position between the loading station and the amplification detection station, and configured to transfer the bin covers (b3) between the reagent bins (a1) and the temporary holding bins (a5) and transfer a sample-containing reagent between the reagent bins (a1) when the transport module (10) moves between the loading station and the amplification detection station; and
an amplification detection module (30), arranged corresponding to the amplification detection station, and configured to perform amplification and detection on a sample solution in the kit (A) when the transport module (10) moves to the amplification detection station.

2. The molecular detection system according to claim 1, wherein the pipetting module (20) is provided with a connecting part configured to connect to or disconnect from a tip (b1), during a process that the transport module (10) moves between the loading station and the amplification detection station, the connecting part is capable of alternatively aligning with the plurality of reagent bins (a1) in a second direction (Y), and the second direction (Y) intersects with the first direction (X);
when the transport module (10) moves until the connecting part aligns with any one of the reagent bins (a1) in the second direction (Y), the pipetting module (20) is controlled to move in the second direction (Y) to drive the connecting part to pick up the bin cover (b3) to open the reagent bin (a1) or release the bin cover (b3) to cover the reagent bin (a1); and
when the transport module (10) moves until the connecting part aligns with any one of the temporary holding bins (a5) in the second direction (Y), the pipetting module (20) is controlled to move in the second direction (Y) to drive the connecting part to release the bin cover (b3) to temporarily hold the bin cover (b3) in the temporary holing bin (a5) or pick up the bin cover (b3) from the temporary holding bin (a5).

3. The molecular detection system according to claim 2, wherein when the transport module (10) moves until the connecting part aligns with any opened reagent bins (a1) in the second direction (Y), and the pipetting module (20) is controlled to move in the second direction (Y) to drive the tip (b1) on the connecting part to be inserted into or withdrawn from a current reagent bin (a1).

4. The molecular detection system according to claim 2, wherein during the process that the transport module (10) moves between the loading station and the amplification detection station, the connecting part is capable of aligning with a tip bin of the kit (A) for pre-installing the tip (b1) in the second direction (Y); and
when the transport module (10) moves until the connecting part aligns with the tip bin in the second direction (Y), the pipetting module (20) is controlled to move in the second direction (Y) to drive the connecting part to be inserted into or withdrawn from the tip bin, to make the connecting part pick up the tip (b1) in the tip bin or release the tip (b 1) on the connecting part to the tip bin.

5. The molecular detection system according to claim 2, wherein during the process that the transport module (10) moves between the loading station and the amplification detection station, the connecting part is capable of alternatively aligning with an injection bin (a3) of the kit (A) and a plunger bin (a2) configured to load a plunger (b2) in the second direction (Y);
when the transport module (10) moves until the connecting part aligns with the plunger bin (a2) in the second direction (Y), the pipetting module (20) is controlled to move in the second direction (Y) to drive the connecting part to be inserted into or withdrawn from the plunger bin (a2) to pick up the plunger (b2); and
when the transport module (10) moves until the connecting part aligns with the injection bin (a3) in the second direction (Y), the pipetting module (20) is controlled to move in the second direction (Y) to drive the plunger (b2) on the connecting part to be inserted into the injection bin (a3) to inject a sample-containing reagent in the injection bin (a3) into a reaction tube (a4) of the kit (A).

6. The molecular detection system according to claim 5, wherein when the transport module (10) moves to the amplification detection station, the reaction tube (a4) of the kit (A) is fitted with and connected to the amplification detection module (30), and the amplification detection module (30) is configured to perform amplification and detection on the sample-containing reagent in the reaction tube (a4) of the kit (A).

7. The molecular detection system according to claim 2, wherein the molecular detection device (1) further comprises a first driving module (40), and the first driving module (40) is in driving connection with the pipetting module (20) to drive the pipetting module (20) to move along the second direction (Y).

8. The molecular detection system according to claim 7, wherein the first driving module (40) comprises a first mounting base (41), a first driving member (42), a driving wheel, a driven wheel (44) and a transmission belt (45); wherein
the pipetting module (20) is movably connected to the first mounting base (41) in the second direction (Y), the first driving member (42) is mounted on the first mounting base (41) and is configured to be electrically connected to a communication module, the driving wheel is in transmission connection with an output shaft of the first driving member (42), the driven wheel (44) is rotatably connected to the first mounting base (41) and is arranged at intervals with the driving wheel in the second direction (Y), and the transmission belt (45) is sleeved between the driving wheel and the driven wheel (44) and is fixedly connected to the pipetting module (20).

9. The molecular detection system according to claim 1, wherein the molecular detection device (1) further comprises a second driving module (50), and the second driving module (50) is in driving connection with the transport module (10) to drive the transport module (10) to move along the first direction (X).

10. The molecular detection system according to claim 9, wherein the second driving module (50) comprises a second mounting base (51), a second driving member (53), a lead screw (52) and a lead screw nut (54); wherein
the transport module (10) is movably connected to the second mounting base (51) in the first direction (X), the lead screw (52) is rotatably connected to the second mounting base (51) around an axis of the lead screw (52), the axis of the lead screw (52) is parallel to the first direction (X), the second driving member (53) is in transmission connection with the lead screw (52) and is configured to be electrically connected to the communication module, and the lead screw nut (54) is threadedly connected to the lead screw (52) and is fixedly connected to the transport module (10).

11. A detection method applied to the molecular detection system according to any one of claims 1 to 10, comprising:
a) moving the transport module (10) to the loading station along a first direction (X), and loading the kit (A) on the transport module (10), wherein the kit (A) is pre-loaded with a sample and various reagents for nucleic acid extraction and pretreatment before detection;
b) moving the transport module (10) to be located between the loading station and the amplification detection station along the first direction (X), and transferring the bin cover (b3) between the reagent bin (a1) and the temporary holding bin (a5) and the sample between each of the reagent bins (a1) through the pipetting module (20), to make the sample be mixed uniformly and reacted with each of the reagents sequentially; and
c) moving the transport module (10) to the amplification detection station along the first direction (X), and performing amplification and detection on a sample-containing reagent in the kit (A) through the amplification detection station.
